# EUROPEAN PATENT SPECIFICATION

(11) **EP 4 196 211 B1**
(45) Date of publication and mention of the grant of the patent: **01.10.2025**
(21) Application number: 21777842.2
(22) Date of filing: 05.08.2021
(51) Int. Cl.: A61N 5/06, A61L 2/08

(54) **LIGHT THERAPY DEVICE**
LICHTTHERAPIEVORRICHTUNG
DISPOSITIF DE LUMINOTHÉRAPIE

(30) Priority: 11.08.2020 HU 2000267
(43) Date of publication of application: 21.06.2023
(73) Proprietor: Zentet-X Zrt., 4225 Debrecen (HU)
(72) Inventor: TÁLAS, László, 4241 Bocskaikert (HU)
(86) International application number: PCT/IB2021/057182
(87) International publication number: WO 2022/034445

(56) References cited:
- EP-A1- 3 222 314
- WO-A1-99/53966
- WO-A2-03/084601
- JP-A- 2006 116 088
- KR-U- 20160 002 747
- US-A1- 2007 219 600
- US-A1- 2012 209 326

## Description

The patent application relates to a light therapy device for the disinfection treatment of fungal, bacterial and viral infections that can be applied in real time to both living and inanimate things, inside and outside the body.

The light therapy device comprises a base unit and a diffuser cap releasably connected to the base unit, the diffuser cap has a reflector element that varies depending on the shape and size of the area to be disinfected. The spectral range of the device is between the light wavelengths of 401 and 490 nanometres, and its light intensity is between 1 and 2000 J/cm2, depending on the area to be disinfected and the degree of infection.

Light therapy has been used in medical technology for decades, and with research showing positive results in more and more fields, it is gaining ground. The light technology device best known to and most widely used by the public is the Bioptron lamp. The Bioptron lamp emits light of a wavelength between 480 and 3400 nm.

A given wavelength of light has a beneficial effect not only on the skin and internal tissues. It has long been known that certain wavelengths of light have a strong fungicidal effect. Ultraviolet radiation (UV-radiation for short), which has shorter wavelengths than visible light (400-780 nm), but longer ones than X-ray radiation (0.01-100 nm), is widely used in medicine; electromagnetic radiation has wavelengths between 180 and 399 nanometres. Strong UV-A radiation (300-350 nm) can directly damage DNA (excites the DNA molecule, rearranging the chemical bonds in the molecule, causing the dimerization of adjacent cytosine bases). Therefore, it is perfectly suitable for the disinfection of devices, however, it can cause skin cancer due to its damaging effects on DNA.

Many solutions are known from the prior art which use the healing power of light of different wavelengths.

Patent No. US 6443978 relates to a device for the physiotherapeutic irradiation of spatially extensive pathologies by light with the help of a matrix of sources of optical radiation such as laser or light diodes. The sources of optical radiation are placed on the surface of a substrate whose shape is adequate to the shape of the zone of pathology, and are connected to a control unit, a power supply unit and a commutation unit. The spectral range covers wavelengths from the ultraviolet range to the radio-wave range.

American Patent No. US 10413624 B2 discloses a portable light fastening assembly for use with the human interface of an electronic device, including a lamp housing containing an UV light source, and an adjustable attachment device suitable for fastening the lamp housing to the interface. A sensor can be included in the germicidal system, wherein the sensor can be in communication with the UV light source, and the sensor can be configured to detect an object proximate to the lamp housing. The germicidal system can also include a processor in communication between the UV light source and the sensor, wherein the processor can be configured to activate the UV light source when the sensor has not detected the object within a first time period, and deactivate the UV light source when the sensor detects the object and a second time period has expired.

The invention disclosed in American patent application No. US20090143842 relates to a phototherapy device to treat infections, diseases and disorders, especially fungal and bacterial nail infections of the hands and feet. This method of treatment is especially important in cases where it is not possible to treat infections with drugs due to the development of drug resistance in microorganisms. The light device contains a source of light. A user can position a hand or foot on a tray to receive a light treatment, and a cover may be used to retain the light from the source within the device and to keep external light from interfering with the treatment.

Patent application No. US20120209326 discloses an UV light pacifier for the treatment of ventilator associated pneumonia (VAP), primarily in the pediatric and neonatal intensive care units of hospitals. The handle of the pacifier includes an UV light assembly, which projects UV light within the mouth of an infant via the pacifier nipple. The UV light assembly has at least one UV light source with at least one associated UV light emitter, which can include an optical fiber, for projecting UV light from the source through the nipple to the surface to be treated.

International patent application No. WO 2011067752 relates to a device for the treatment of rhinitis by biostimulative illumination, comprising a pair of LEDs containing probes adapted to be inserted into the nostrils of a patient, and the probes are supported by a probes support casing. The casing accommodates an electric power source and a micro-switch for activating/deactivating the LEDs. The micro-switch becomes activated by pressing against a side-wing of the casing.

Patent No. EP2545962 discloses an irradiation device for insertion into an orifice of the human body for providing photodynamic therapy, the orifice being the female reproductive tract or the rectum, and the device comprises a housing adapted to be fully inserted and secured in the orifice, the housing enclosing a LED lamp system and a power source. The housing comprises a treatment surface with the LED system, which is at least partially transparent to light when the device is in operation. The treatment surface has a size and/or shape adapted for complementary fit with a treatment area within the orifice. The invention is characterized in that the housing has a flexible outer portion that can adjust its shape to form a secure fit with the walls of the orifice, and the LED system is arranged to emit light at a light intensity in the range of 1-50 mW/cm2. A transparent material is used to form both the housing around the LED lamp system and also the treatment surface, thereby acting as both a housing for the LED lamp system and a diffuser for the light.

Patent application No. US20090088824 discloses a light therapy device including multiple light-emitting diodes (LEDs) positioned in a handheld portable device. The housing and the LEDs are configured to have direct contact with the skin or tissue of the user without any intermediary materials, and light the surface and underlying layers of tissue for photodynamic stimulation of the cells. Two iterations of the device utilize light known to have a bactericidal effect in the case or acne or Rosacea. The devices are fabricated from an injection molded plastic housing. The housing contains an arrangement of 36-72 LEDs on a circuit board in an arrangement to provide even lighting over the skin or tissue surface.

The invention disclosed in Hungarian patent publication No. P9903603 provides an improved balloon catheter apparatus for use in therapies requiring the delivery of uniform light to a treatment area. The improved apparatus comprises a balloon having a defined treatment window where the window is delineated using a reflective material. The apparatus may further include a fiber optic cable that terminates in a diffusion tip where the diffusion tip is longer than the treatment window.

A light therapy device for disinfection treatment is also known from WO 03/084601 A2.

A disadvantage of the known solutions is that the devices making use of the photodynamic effect act on only one area to be treated/disinfected, and use mostly UV radiation, there is no solution that would be suitable for disinfecting both living and non-living things, inside and outside the body.

The aim of the invention is to develop a device that uses light of wavelengths between 401 and 490 nm and is suitable for disinfection against fungal, bacterial and viral infections (e.g.: *Candida albicans, Candida grablata, Staphylococcus aureus, Propionibacterium acnes, Enterococcus faecalis, Porphyromonas gingivalis, Fusobacterium nucleatum, Trichophyton rubrum, Trichophyton mentagrophytes, Aspergillus niger,* blue rot (*Penicillium italicum*), green rot (*Penicillium digitatum*), grey mould (*Botrytis cinerea*) and stem end rot (*Phomopsis citri*), and various viruses), and can be applied in real time to both living and inanimate things, either inside or outside the body.

The aim is to develop a device - comprising a base unit provided with a diffuser cap - that is suitable for versatile use, is easy and safe to use, and is low cost.

The invention is defined in the appended claims.

To achieve a targeted effect, the base unit requires an appropriate diffuser cap and a light source emitting light of an appropriate wavelength. The diffuser cap has a reflector element of varying shape, preferably adapted to the shape of the area to be treated, and thus an even more effective disinfection effect can be achieved.

The idea of the invention is based on the fact that visible light (especially in the violet-blue region of the spectrum, between 401 and 490 nm) alone can kill bacteria, fungi and viruses. This invention uses wavelengths between 401 and 490 nanometres which, although have a lower energy than UV radiation, still have a disinfecting effect, but they cannot penetrate the DNA, so they do not damage it.

The mechanism used is essentially the natural accumulation of photoactive porphyrins, such as uroporphyrin, coproporphyrin and to a lesser extent protoporphyrin. Due to their strong absorption and biological significance, porphyrin compounds are widely studied compounds in photochemistry. Porphyrins are well-known, naturally occurring compounds composed of four pyrrole rings and methine bridges connecting the rings. Metal porphyrins are vital compounds in the living world. Light is absorbed by the Soret band of porphyrins and converted to a triplet state, eventually creating reactive oxygen species that damage bacteria and viruses, and this leads to their destruction.

The light therapy device developed for achieving the set aim comprises a base unit and a diffuser cap connected to the base unit.

The housing of the base unit consists of a closed inner housing and an outer housing surrounding it. There is at least one light source on the upper side of the housing, on the outside. In the inner housing there is a power supply unit and a control unit (driver), and a switch is positioned on the lower part of the housing, breaking through the walls of both the inner housing and the outer housing.

The light source has two electrical wires, a short leg connected to the power supply unit and a long leg ending in the inner housing above the switch and under the control unit. The long leg is insulated with an insulating element above the power supply unit.

There is a connecting portion on both sides of the outer housing, and these connecting portions are used for connecting the diffuser cap.

The main parts of the diffuser cap are: a reflector element, a connecting element and a locking element. The reflector element is positioned above the light source, directing its light to the area to be treated, its shape is adapted to the area to be treated.

The connecting element is connected to the lower part of the reflector element, it can be formed in the continuation of the lower part of the reflector element, but it can also be an element fixed to the lower part of the reflector element, made of the material of the reflector or some other material. The connecting element is positioned on the outer housing of the base unit and, depending on the intended use of the diffuser cap, it can cover a part of the base unit, or have a stem-like design. The end of the connecting element is provided with a locking element comprising at least one pin.

The connecting portion has an L-shaped slot having a vertical opening, a vertical arm, and a horizontal opening, a horizontal arm, the two openings are the continuation of each other, their shape matches the shape of the pin(s) of the locking element, and their width, length and depth matches the width, length and depth of the pin(s) of the locking element, thus ensuring a perfect fit.

The diffuser cap is fixed to the base unit by inserting and sliding the pin of the locking element in the vertical arm of the L-shaped slot of the connecting portion, then by turning the diffuser cap and the base unit in the opposite direction, the pin of the locking element is displaced, slides into the horizontal arm of the L-shaped slot of the connecting portion, thus the diffuser cap is fixed firmly to the base unit.

This solution provides a releasable connection between the base unit and the diffuser cap. The base unit and the diffuser cap are separated by pressing the pin of the locking element and turning it in the direction opposite to that of fixing, then the locking element pops out from the connecting portion of the base unit, and the diffuser cap can be detached.

By detaching the diffuser cap, the base unit can be removed and serviced (battery, light source replacement), or the diffuser cap can be transferred to another base unit.

Depending on the intended use of the light therapy device, there may be an additional element between the reflector element and the connecting element. The purpose of the additional element is either to make it look more similar to an object used in everyday life, or to ensure a better fit for the reflector element.

The material of the inner housing and the outer housing can vary depending on the treatment area, it can be e.g. medical silicone, plastic, or metal, and its shape shall provide a secure fit for the shape of the diffuser cap and the reflector element to be connected, it can be e.g. rectangular, square or rounded. The outer housing is preferably made of a waterproof material. The material of the locking element is a material suitable for sterilization and locking, it can be metal, alloy, or high-purity medical plastic.

The light source can be any diode emitting light of a wavelength having a therapeutic effect, preferably a LED (Light-Emitting Diode), one base unit may comprise one or more light sources. The wavelength of the light used for irradiation shall be selected from the range between 401 nm and 490 nm to obtain an effective photodynamic effect, thus the light sources chosen shall be capable of emitting light of a wavelength and intensity appropriate for this purpose.

The power supply unit supplies appropriate power. The source of power can be any energy storage device that during the charging process converts the supplied electricity into chemical energy, can store it for a longer period of time in the form of chemical energy, and during the discharging process converts it back into electricity. The source of power is suitable directly only for storing and supplying direct voltage. Accordingly, the source of power can be (one or more) batteries (rechargeable or regular), or transformed direct current.

The batteries shall be operated in such a way that the electrochemical reactions do not present a hazard in the case of human photodisinfection inside the body. The batteries can be lithium batteries, or equivalent batteries with sufficient capacity that can also be stored for up to 10 years. They can be regular batteries or batteries rechargeable using a closed charging system. The power supply unit, regardless of its material, is located inside the housing and does not come into direct contact with the outside world. Sealing is provided to prevent fluids from leaking into or out of the device. The simplest way to connect a *light source* to the power supply is through electrical connections. The batteries can be rechargeable or regular. Optionally, the battery can have an input port for connecting a cable. In such embodiments, the input port is configured to receive an USB or other plug connector. The input port shall be sealed when the device is in use.

The switch can be a micro-switch, single-phase or two-phase, positioned on the lower part of the housing, and it is switched on by fully depressing and then releasing it (click), thereby the circuit of the base unit is closed.

An insulating element is used to insulate the electrical wires (legs) of the light source to prevent an electrical short circuit. It can be made of any insulating material that is not harmful to human health or the device.

The diffuser cap is a refractive cap that can be positioned and fixed over the light source. Its use increases the amount of scattered light. An appropriately shaped diffuser cap brings light closer to the area to be disinfected, thus providing a more targeted effect. The reflector element of the diffuser cap can be made of any light-scattering material that is not harmful to human health or the base unit. The shape of the diffuser cap and the reflector element connected to the base unit of the light therapy device is determined by the area of application.

The device according to the invention is controlled by a control unit (driver). Its role is to operate and program the light source. The control unit controls the amount of electricity supplied to the light source, and the type and number of lighting modes of the light source. The driver also monitors the power supply unit and the light source, it has an over-discharge protection circuit and a temperature sensor, so before the light source overheats, it automatically lowers the light intensity to reduce the heat load. Its most important physical characteristic is its diameter, as this determines the shape of the housing into which it can be easily installed without alteration, that is the area of therapy where it can be applied. The control unit may also include a microcontroller and a microprocessor for digital control and development. (E.g. for programming a delay, the duration of treatment, the number and length of light pulses, diagnosis from other measuring instruments, etc.). Alternatively, the program commands may be transmitted to the device by means of a wireless connection, for example, the receiver may be an infra-red or radio wave receiver.

Light therapy devices suitable for disinfecting living things can be used either inside living a body, then the diffuser cap and reflector element of the light therapy device may be similar to objects used in everyday life, e.g. pacifiers, suppositories, tampons, nasal plugs that can be inserted into the orifices of the body to be treated (eye, mouth, ear, nose, vagina, rectal orifice), or outside a living body, e.g. devices used for disinfecting fodder.

Another major group of light therapy devices is suitable for disinfecting inanimate things inside a living body, and can be used for disinfecting objects used in everyday life. Such objects are mainly fluid inlet and outlet devices used in healthcare (e.g. permanently inserted plastic and silicone devices, catheters, branulas, cannulas, dental implants, dentures). The reflector element used for the disinfection of these objects, for the destruction of bacteria and fungi adhering to the devices, preferably consists of optical light cables, light tubes.

The third major group of light therapy devices is suitable for disinfecting inanimate things outside a living body, and can be used for disinfecting objects used in everyday life that come into direct contact with humans. Such objects include e.g. phone cases, watches, laptops, computers, tablets, etc.

The device according to the invention is described in detail through embodiments with reference to the following figures, without being limited thereto:
Figure 1 shows a cross-sectional view of the base unit of the light therapy device according to the invention;
Figure 2 shows a longitudinal sectional view of a light therapy device that can be inserted into the vagina;
Figure 3 shows a longitudinal sectional view of a light therapy device that can be inserted into the mouth;
Figure 4 shows a longitudinal sectional view of a light therapy device suitable for disinfecting catheters;
Figure 5 shows a longitudinal sectional view of a light therapy device suitable for disinfecting fodder.

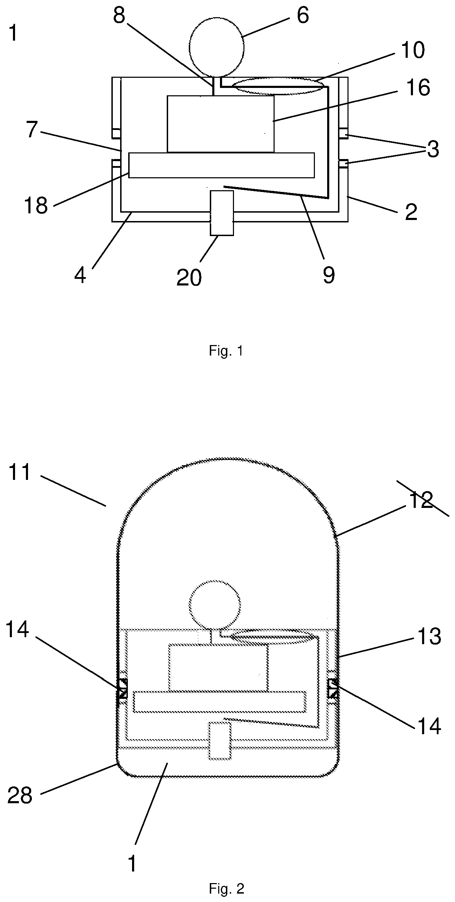

Figure 1 shows the base unit 1 of the light therapy device. The sides and the lower part of an inner housing 4 are surrounded by an outer housing 2. A light source 6 is connected to the upper side of the inner housing 4. The light source 6 is provided with two electrical wires, a short leg 8 and a long leg 9. The short leg 8 is connected directly to a power supply unit 16. The electrical wires are separated from each other by insulation 10.

A control unit 18 is positioned under the power supply unit 16, and a switch 20 is positioned by breaking through the walls of both the outer housing 2 and the inner housing 4. The long leg 9 ends above the portion of the switch 20 protruding into the inner housing 4, and when the device is not in operation, it does not come into contact with either the switch 20 or the control unit 18.

There is an L-shaped connecting portion on both sides of the outer housing 2, consisting of a horizontal arm 3 and a vertical arm 7.

The embodiment shown in Figure 2 comprises a base unit 1 provided with a diffuser cap 11 that can be inserted into the vagina. The reflector element 12 of the diffuser cap 11 has a rounded design, a connecting element 13 is connected to the lower part of the reflector element 12, in the continuation thereof, on both sides of the outer housing 2. The end of the connecting element 13 is provided with a locking element 14 that connects to the connecting portion of the base unit 1. The locking element 14 comprises a pin.

The reflector element 12 covering the light source 6 has a rounded design to fit as precisely as possible into the target area. In this embodiment, for hygienic reasons, the lower part of the base unit 1 is also surrounded by a protective part 28 made of the same material as the reflector element 12. The protective part 28 is also fixed to the connecting portion by locking elements 14 comprising a pin.

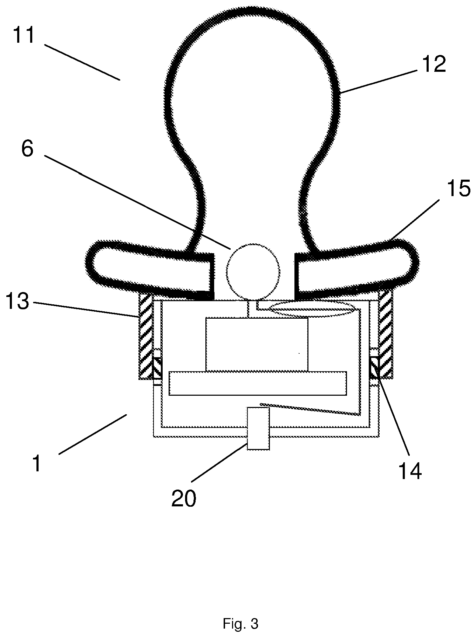

In Figure 3, the reflector element 12 of the diffuser cap 11 fixed to the base unit 1 is similar to the nipple of a pacifier. Apparently, in this embodiment, the device according to the invention resembles a commonly used pacifier, but the usual handle of the pacifier is replaced by the base unit 1. The reflector element 12 is fixed to an additional element 15, which is similar to the shield portion of a pacifier. Connecting elements 13 having a stem-like design and provided with a locking element 14 comprising two pins are connected to the lower part of the additional element 15, in the continuation thereof.

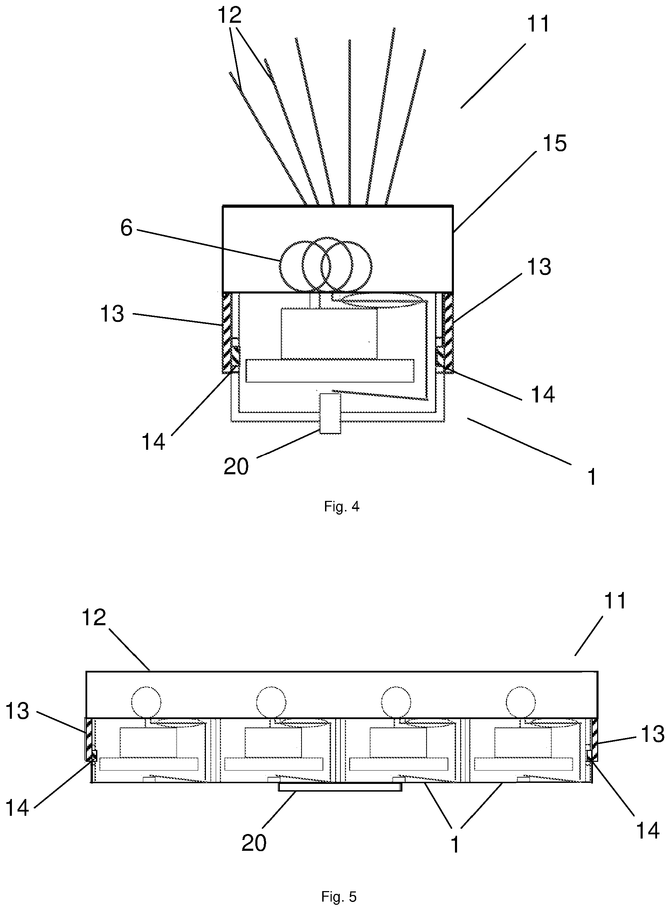

The light therapy device shown in Figure 4 is suitable for disinfecting catheters. In this embodiment, the reflector element 12 of the diffuser cap 11 consists of light cables transmitting light, which are fixed in an additional element 15 above the light sources 6. The additional element 15 completely covers the upper part of the base unit 1, and is provided with connecting elements 13 connected to the lower part of the additional element 15. For the disinfection of catheters, the reflector elements 12 consisting of light cables are introduced into the catheters.

The light therapy device shown in Figure 5 can be used for disinfecting fodder. Then, since the area to be disinfected is large, preferably multiple base units 1 are connected to each other in series. In the embodiment shown, the base units 1 have one light source 6 each, which face in one direction, and are provided with only one switch 20. In this embodiment, there is a single diffuser cap 11, the reflector element 12 of which covers the light source 6 of each base unit 1.

The operation of the light therapy device is simple. A base unit 1 provided with an appropriate diffuser cap 11 is placed on the area to be disinfected, and switched on by pressing the switch 20, then the switch 20 connects the long leg 9 to the control unit 18.

During the treatment, the disinfection mechanism is essentially the natural accumulation of photoactive porphyrins such as uroporphyrin, coproporphyrin, and to a lesser extent protoporphyrin. Due to their strong absorption and biological significance, porphyrin compounds are widely studied compounds in photochemistry. Light is absorbed by the Soret band of porphyrins and converted to a triplet (excited) state, leading to the formation of singlet oxygen. The released oxygen free radical is highly toxic and oxidizing, and can therefore cause damage to the cell membranes and nucleic acids of pathogens. This leads to the complete destruction of pathogens, however, light of a wavelength between 401 and 490 nm, due to its wave nature, does not reach the DNA in the deeper layers of the human body, therefore does not damage it.

Treatment with the base unit provided with different diffuser caps is described in detail through the following examples:
In Examples 1 to 3 it is used against the pathogens *Candida albicans, Staphylococcus aureus, Acinetobacter baumannii* and *Pseudomonas aeruginosa, Trichophyton rubrum, Trichophyton mentagrophytes, Aspergillus niger,* etc.:

### Example 1: a device suitable for disinfecting the vagina

In this example, the diffuser 11 has a rounded, conical design to best fit the shape of the surface to be disinfected, the vagina.

Using a light therapy device of this design, the inner surface of the vagina is exposed to radiation at a wavelength of 415 nm and an intensity of 1 J/cm2 to 432 J/cm2, preferably 432 J/cm2. The duration of irradiation is 5 to 20 minutes/day, preferably 20 minutes/day. Irradiation at a lower light intensity can be compensated by a longer irradiation time.

### Example 2: a device suitable for disinfecting the mouth

Infant oral thrush (in Latin: *soor oris*) feared by many mothers is nothing more than a fungal infection caused by the fungus *Candida albicans. Soor oris* usually occurs in infants under the age of six months, but it can also occur after the age of six months, in the latter case mainly the weakening of the immune system of the body for various reasons or antibiotic treatment leads to the growth of fungi on the oral mucosa. As *Candida albicans* infection spreads in the mouth, saliva, acting as a carrier of the infection, can infect virtually anything it comes into contact with. Although oral thrush is not dangerous, it can be a concern due to increased sensitivity or pain. Using a base unit 1 provided with a pacifier shaped reflector element 12, the inner surface of the mouth is exposed to radiation at a wavelength of 405 to 420 nm and an intensity of 1 J/cm2 to 320 J/cm2, preferably 320 J/cm2. The duration of irradiation is 5 to 20 minutes/day, ideally 20 minutes/day. Irradiation at a lower light intensity can be compensated by a longer irradiation time.

### Example 3: a device suitable for disinfecting catheters

In this example, the reflector element 12 of the diffuser 11 consists of light cables, which are introduced into the catheters. Preferably, they can be used for disinfecting the outer surface of the catheters. Using a light therapy device, the inner surface of the catheters is exposed to radiation at a wavelength of 405 nm and an intensity of 47.4 J/cm2 to 284.4 J/nm2, preferably 284.4 J/cm2. The duration of irradiation is 5 to 30 minutes/day, ideally 30 minutes/day. Irradiation at a lower light intensity can be compensated by a longer irradiation time.

### Example 4: a device suitable for disinfecting fodder

In this example, the light therapy device is used for disinfecting fodder against common plant pathogens, blue rot (*Penicillium italicum*), green rot (*Penicillium digitatum*), and grey mould (*Botrytis cinerea*). Base units 1 connected in series are placed next to or above the plant surface to be treated, at a distance of 5 to 30 cm, exposing it to radiation at a wavelength of 410 nm and an intensity of 70 J/cm2 to 320 J/cm2, preferably 320 J/cm2. The duration of irradiation is 5 to 20 minutes/day, ideally 20 minutes/day. Irradiation at a lower light intensity can be compensated by a longer irradiation time.

A further advantage of the solution according to the invention is that therapy with the light therapy device requires no added photosensitizer, does not use potentially harmful ultraviolet radiation, and kills microbial cells, regardless of their antibiotic resistance status.

## Claims

1. A light therapy device for the disinfection treatment of fungal, bacterial and viral infections, comprising a base unit (1) and a diffuser cap (11) provided with a reflector element (12) connected to the base unit (1), where the base unit (1) has an inner housing (4), to the upper side of which at least one light source (6) is connected,
the light emitted by the light source (6) is in the wavelength range of 401 to 490 nm,
in the inner housing (4) there is a power supply unit (16), under which there is a control unit (18), the control unit (18) includes a microcontroller and a microprocessor,
a switch (20) is positioned on the lower part of the inner housing (4), breaking through the wall thereof; the light source (6) has two electrical wires: a short leg (8), which is connected to the power supply unit (16), and a long leg (9), which ends between the switch (20) and the control unit (18), the long leg (9) is provided with an insulating element (10), the inner housing (4) is surrounded by an outer housing (2), on the outer housing (2) there are at least two connecting portions,
wherein the connecting portions include an L-shaped slot having a vertical arm (7) and a horizontal arm (3), the vertical arm (7) and the horizontal arm (3) are the continuation of each other; the diffuser cap (11) also includes connecting elements (13) connected to the lower part of the reflector element (12), and the connecting elements (13) are provided with a locking element (14) comprising at least one pin, the locking element (14) fixes the diffuser cap (11) to the connecting portion of the base unit (1).

2. The light therapy device according to claim 1, **characterized in that** there is an additional element (15) between the reflector element (12) and the connecting elements (13) of the diffuser cap (11).

3. The light therapy device according to claim 1, **characterized in that** the light source (6) is an LED.

4. The light therapy device according to any of claims 1 to 3, **characterized in that** the reflector element (12) is made of medical silicone.

5. The light therapy device according to any of claims 1 to 4, **characterized in that** the reflector element (12) consist of light cables.

6. The light therapy device according to claim 5, **characterized in that** the light cables are fixed in the additional element (15) of the diffuser cap (11).

## Patentansprüche

1. Lichttherapievorrichtung zur desinfizierenden Behandlung von Pilz-, Bakterien- und Virusinfektionen, umfassend eine Basiseinheit (1) und eine mit einem Reflektorelement (12) versehene Diffusorkappe (11), die mit der Basiseinheit (1) verbunden ist, wobei die Basiseinheit (1) ein Innengehäuse (4) aufweist, an dessen Oberseite mindestens eine Lichtquelle (6) angeordnet ist,
das von der Lichtquelle (6) emittierte Licht im Wellenlängenbereich von 401 bis 490 nm liegt,
im Innengehäuse (4) eine Stromversorgungseinheit (16) angeordnet ist, unter der sich eine Steuereinheit (18) befindet, wobei die Steuereinheit (18) einen Mikrocontroller und einen Mikroprozessor umfasst,
ein Schalter (20) am unteren Teil des Innengehäuses (4) angeordnet ist, der dessen Wand durchbricht; die Lichtquelle (6) zwei elektrische Drähte aufweist: einen kurzen Schenkel (8), der mit der Stromversorgungseinheit (16) verbunden ist, und einen langen Schenkel (9), der im Bereich zwischen dem Schalter (20) und der Steuereinheit (18) endet, wobei der lange Schenkel (9) mit einem Isolierelement (10) versehen ist, das Innengehäuse (4) von einem Außengehäuse (2) umgeben ist, am Außengehäuse (2) mindestens zwei Anschlussabschnitte vorhanden sind,
wobei
die Verbindungsabschnitte einen L-förmigen Schlitz mit einem vertikalen Arm (7) in den horizontalen Arm (3) übergeht, wobei der vertikale Arm (7) und der horizontale Arm (3) die Fortsetzung voneinander sind; die Diffusorkappe (11) auch Verbindungselemente (13) umfasst, die mit dem unteren Teil des Reflektorelements (12) verbunden sind, und die Verbindungselemente (13) mit einem Verriegelungselement (14) versehen sind, das mindestens einen Stift umfasst, wobei das Verriegelungselement (14) die Diffusorkappe (11) an dem Verbindungsabschnitt der Basiseinheit (1) befestigt.

2. Lichttherapievorrichtung nach Anspruch 1, **dadurch gekennzeichnet, dass** zwischen dem Reflektorelement (12) und den Verbindungselementen (13) der Diffusorkappe (11) ein zusätzliches Element (15) vorhanden ist.

3. Lichttherapievorrichtung nach Anspruch 1, **dadurch gekennzeichnet, dass** die Lichtquelle (6) eine LED ist.

4. Lichttherapievorrichtung nach einem der Ansprüche 1 bis 3, **dadurch gekennzeichnet, dass** das Reflektorelement (12) aus medizinischem Silikon hergestellt ist.

5. Lichttherapievorrichtung nach einem der Ansprüche 1 bis 4, **dadurch gekennzeichnet, dass** das Reflektorelement (12) aus Lichtkabeln besteht.

6. Lichttherapievorrichtung nach Anspruch 5, **dadurch gekennzeichnet, dass** die Lichtkabel in dem Zusatzelement (15) der Diffusorkappe (11) befestigt sind.

## Revendications

1. Dispositif de luminothérapie pour le traitement désinfectant d'infections fongiques, bactériennes et virales, comprenant une unité de base (1) et un capot diffuseur (11) muni d'un élément réflecteur (12) relié à l'unité de base (1), l'unité de base (1) comportant un boîtier intérieur (4) sur la face supérieure duquel est reliée au moins une source lumineuse (6),
la lumière émise par la source lumineuse (6) se situe dans la gamme de longueurs d'onde de 401 à 490 nm,
dans le boîtier intérieur (4) se trouve une unité d'alimentation électrique (16), sous laquelle se trouve une unité de commande (18), l'unité de commande (18) comprend un microcontrôleur et un microprocesseur,
un interrupteur (20) est positionné sur la partie inférieure du boîtier intérieur (4),
traversant la paroi de celui-ci ; la source lumineuse (6) comporte deux fils électriques : une branche courte (8), qui est reliée à l'unité d'alimentation électrique (16), et une branche longue (9), qui se termine entre l'interrupteur (20) et l'unité de commande (18), la branche longue (9) est pourvue d'un élément isolant (10), le boîtier intérieur (4) est entouré d'un boîtier extérieur (2), sur le boîtier extérieur (2) se trouvent au moins deux parties de raccordement,
dans lequel
les parties de raccordement comprennent une fente en forme de L ayant un bras vertical (7) et un bras horizontal (3), le bras vertical (7) et le bras horizontal (3) se prolongeant l'un l'autre ; le capuchon diffuseur (11) comprend également des éléments de raccordement (13) reliés à la partie inférieure de l'élément réflecteur (12), et les éléments de raccordement (13) sont pourvus d'un élément de verrouillage (14) comprenant au moins une goupille, l'élément de verrouillage (14) fixant le capuchon diffuseur (11) à la partie de raccordement de l'unité de base (1).

2. Dispositif de luminothérapie selon la revendication 1, **caractérisé en ce qu'**il existe un élément supplémentaire (15) entre l'élément réflecteur (12) et les éléments de raccordement (13) du capuchon diffuseur (11).

3. Appareil de luminothérapie selon la revendication 1, **caractérisé en ce que** la source lumineuse (6) est une LED.

4. Appareil de luminothérapie selon l'une quelconque des revendications 1 à 3, **caractérisé en ce que** l'élément réflecteur (12) est en silicone médical.

5. Appareil de luminothérapie selon l'une quelconque des revendications 1 à 4, **caractérisé en ce que** l'élément réflecteur (12) est constitué de câbles lumineux.

6. Appareil de luminothérapie selon la revendication 5, **caractérisé en ce que** les câbles lumineux sont fixés dans l'élément supplémentaire (15) du capuchon diffuseur (11).
